# EUROPEAN PATENT APPLICATION

(11) **EP 4 786 481 A1**
(43) Date of publication of application: **05.08.2026**
(21) Application number: 23957710.9
(22) Date of filing: 02.11.2023
(51) Int. Cl.: C07K 1/14

(54) **PROTEIN PURIFICATION DEVICE AND METHOD FOR PRODUCING PURIFIED PROTEIN**

(71) Applicant: Noritake Co., Limited, Nagoya-shi, Aichi 451-8501 (JP)
(72) Inventor: KUMAGAI, Kazuaki, Nagoya-shi, Aichi 451-8501 (JP)
(74) Representative: Kramer Barske Schmidtchen Patentanwälte PartG mbB
(86) International application number: PCT/JP2023/039721
(87) International publication number: WO 2025/094385

(57) **Abstract**

Provided is a technology for more efficiently producing purified protein from a cell culture liquid. A device disclosed herein is for purifying protein from a cell culture liquid. The device comprises a hydrocyclone, a first reservoir tank for retaining a first liquid, a first pump, and a second pump. The first pump is provided at a site where the flow rate of liquid discharged from the hydrocyclone can be adjusted. The second pump is provided at a site where the flow rate of the first liquid discharged from the first reservoir tank can be adjusted.

## Description

### [Technical Field]

A present disclosure relates to a protein purification device and a method for producing the purified protein.

### [Background Art]

International Patent Publication 2019/189064 discloses a device that uses a module, in which an inline mixer and a cyclone are bound, so as to continuously purify proteins from a cell culture liquid. This device is configured with a first module and a second module. In the first module, a pipe linking a culture tank or a cell separation device with the first module and a pipe linking a first reservoir tank with the first module are connected through a branch valve to an inlet port of the inline mixer. An outlet port of the inline mixer is connected to an inlet port of a hydrocyclone. To a lower part outlet port of the hydrocyclone, a pipe linking a first reaction module with a second module is bound. To an upper part outlet port of the hydrocyclone, a pipe is bound. In the second module, a pipe linking the first module with the second module and a pipe linking a second reservoir tank with the second module are connected through a branch valve to the inlet port of the inline mixer. The outlet port of the inline mixer and the inlet port of the hydrocyclone are connected. To an outlet port at a hydrocyclone lower part, a pipe linking the second module with another module is bound. To a hydrocyclone upper part outlet port, a pipe is bound. This publication describes that, by using the device including the above described configuration, it is possible, even with a sample containing a small amount of cells and cell debris, host microorganisms, or the like, under a suspended state, to continuously collect and purify target proteins from the sample in a short time.

Japanese Patent Application Publication No. 2000-509325 discloses a method that includes steps of (a) to (d) so as to separate an emulsion into a water-miscible phase and a water-immiscible phase, although the emulsion contains the water-miscible phase, the water-immiscible phase, and a fine solid particle. The step (a) is a step for introducing the emulsion to a first hydrocyclone so as to separate the emulsion into an overflow emulsion and an underflow emulsion. The step (b) is a step for reversing a phase of the overflow emulsion. The step (c) is a step for introducing the overflow emulsion to another next hydrocyclone or other next hydrocyclones arranged in series. The step (d) is a step for collecting the water-miscible phase and the water-immiscible phase, and collecting the solid particle in a discontinuous phase of the overflow emulsion at the step (c). This publication describes that it is possible, by using the method that includes the above described configuration, to collect a high purity oil at a high yield from the emulsion being oil/water/biocatalyst of a water continuous system, although the emulsion would be generated by a biological desulfurization method.

### [Citation List]

### [Patent Document]

[Patent Document 1] International Patent Publication 2019/189064
[Patent Document 2] Japanese Patent Application Publication No. 2000-509325

### [Summary of Invention]

### [Technical Problem]

The present inventor thinks to use the cell culture liquid so as to further efficiently produce the purified protein.

### [Solution to Problem]

According to a herein disclosed technique, a device is disclosed that purifies a protein from a culture liquid of a cell. This device includes a hydrocyclone, a first reservoir tank that retains a first liquid, a first pump, and a second pump. The first pump is provided at a site where a flow rate of a liquid discharged from the hydrocyclone can be adjusted. The second pump is provided at a site where a flow rate of the first liquid discharged from the first reservoir tank can be adjusted. By using the device that includes the configuration as described above, it is possible with the cell culture liquid to further efficiently produce the purified protein.

The device might further include an inline mixer, a first liquid delivery line, and a second liquid delivery line. The inline mixer might be positioned at a downstream of the hydrocyclone. The first liquid delivery line might connect the hydrocyclone and the inline mixer. The second liquid delivery line might extend from the first reservoir tank and be joined with the first liquid delivery line. On the first liquid delivery line, the first pump might be provided at a at a position closer to the hydrocyclone than to a confluence site joined with the second liquid delivery line. On the second liquid delivery line, the second pump might be provided at at a position closer to the first reservoir tank than to the confluence site joined with the first liquid delivery line.

The hydrocyclone might be configured to separate a liquid, which has been sent to the hydrocyclone, into an A liquid and a B liquid. The hydrocyclone might include a first derivation port that derives the A liquid and include a second derivation port that derives the B liquid. The first derivation port might be connected to the first liquid delivery line. The first pump might be provided between the first derivation port and the confluence site joined with the second liquid delivery line.

The device might be used for purifying an antibody produced from a cultured cell.

The protein being a purification target might be an antibody. An antibody binding protein, which is a ligand, might be used for purifying the antibody.

According to the herein disclosed technique, a method for producing the purified protein is disclosed. This producing method includes purifying the protein from the culture liquid of the cell, while using the above described device. By using the producing method that includes the configuration as described above, it is possible with the cell culture liquid to further efficiently produce the purified protein.

### [Brief Description of Drawings]

FIG. 1 is a schematic view of a device 100.
FIG. 2 is a schematic view of a device 1.
FIG. 3 is an enlarged schematic view of the device 1.
FIG. 4 is an enlarged schematic view of the device 1.

### [Description of Embodiments]

Below, a preferred embodiment of the herein disclosed technique would be explained. Incidentally, the matters other than matters particularly mentioned in this description and required for practicing the present disclosure can be understood on the basis of a technical content taught by the present description and a common general technical knowledge of a person skilled in the art. The herein disclosed technique can be implemented on the basis of a content disclosed in the present description and a common general technical knowledge in this technical field. Incidentally, a wording of A to B (A and B are arbitrary values) in the present description and in claims means being equal to or more than A and not more than B, and in addition, semantically covers a situation of being more than A and less than B.

According to the technique disclosed herein, a device is disclosed which purifies a protein from a culture liquid of a cell. In the present description, a wording "cell" means a cell that produces a protein being a purification target and that can be cultured outside a living organism (in vitro). The "cell" contains, for example, a cell line and a cell mass, tissue, or organ which is removed from a living organism. The wording "cell line" means a clone of a primary cell that can be stably cultured in vitro for many generations. As the cell, it is possible to use, for example, a microbial cell, such as E. coli; yeast; an insect cell, such as S2 cell, Sf9 cell, and Sf21 cell; and a mammal cell, such as COS cell, NS0 cell, Sp2/0 cell, CHO cell, HEK cell, HeLa cell, and hybridoma. As the cell, for example, the mammal cells can be preferably used, and among them, a NS0 cell, a Sp2/0 cell, a CHO cell, a HEK cell, a hybridoma, and the like are preferably used. The cell might be a cell that is genetically modified to produce the protein being the purification target. In the present description, the wording "culture liquid" means a culture liquid being in a state where the above described cell has been cultured and the protein being the purification target has been produced and secreted.

The protein being the purification target for the herein disclosed technique is, for example, a protein that can be used as a medicine. As the protein, it would be, for example, an antibody; a hematopoietic protein, such as erythropoietin; a cytokine, such as interferon α, interferon β, interferon γ, G-CSF, and GM-CSF; an enzyme, such as t-PA; a hormone, such as insulin, human growth hormone, and estrogen; or the like. Among them, it is preferable that the protein is the antibody. As the antibody, it is good to be the monoclonal antibody, it might be, for example, a mouse antibody or a chimeric antibody, but it is preferable to be a humanized antibody, or a fully humanized antibody. An antigen recognized by the antibody is not particularly restricted, but suitably set according to an object.

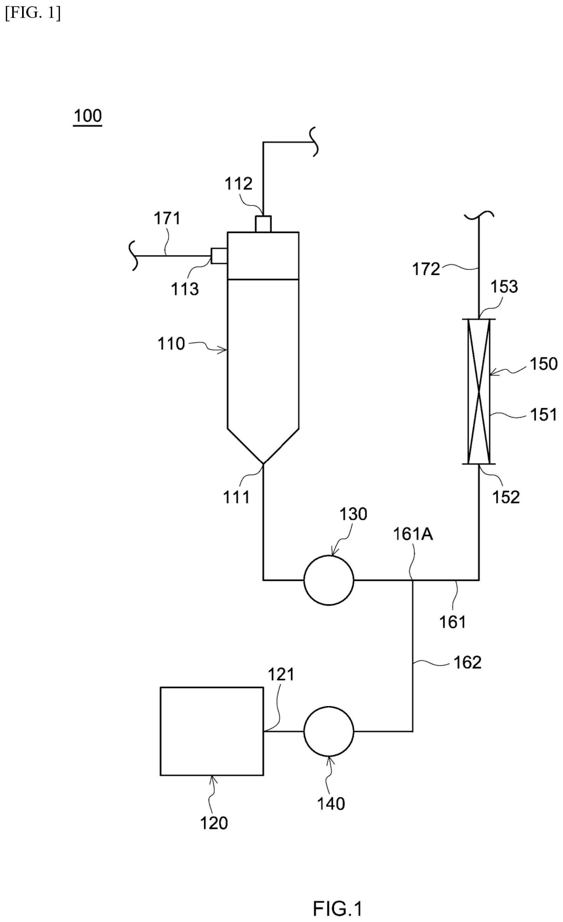

FIG. 1 is a schematic view of a device 100. As shown in FIG. 1, the herein disclosed device 100 includes a hydrocyclone 110, a first reservoir tank 120 configured to retain a first liquid, a first pump 130, and a second pump 140. The first pump 130 is provided at a site where a flow rate of the liquid discharged from the hydrocyclone 110 can be adjusted. The second pump 140 is provided at a site where a flow rate of the first liquid discharged from the first reservoir tank 120 can be adjusted. Incidentally, although omitted in FIG. 1, the device 100 might suitably include a valve, a pressure gauge, a flow meter, and the like, as needed.

The hydrocyclone 110 is configured, for example, to separate a liquid, which has been sent to the hydrocyclone 110, into A liquid and B liquid. As shown in FIG. 1, the hydrocyclone 110 is formed in a funnel shape, and includes a first derivation port 111 at a lower end part, a second derivation port 112 at a top end part, and an inlet port 113 at a side surface of an upper side. The first derivation port 111 is, for example, a site where the A liquid is derived from the hydrocyclone 110. In the embodiment shown by FIG. 1, the first derivation port 111 is connected to a first liquid delivery line 161. The second derivation port 112 is a site where the B liquid is derived from the hydrocyclone 110. The second derivation port 112 is, for example, connected to a drainage line outside the device 100, or to another liquid delivery line. The inlet port 113 is a site where the liquid having been sent from the upstream is introduced in the hydrocyclone 110. In this embodiment, the inlet port 113 is connected to a pipe 171. In this embodiment, at an upstream of the hydrocyclone 110, a supply source that supplies a liquid to the hydrocyclone 110 (for example, reservoir tanks for various liquids, a cell culture tank, or the like) is arranged. It is good that the pipe 171 is configured to directly or indirectly connect the above described supply source and the hydrocyclone 110. Between the supply source and the hydrocyclone 110, it is good to arrange, for example, the inline mixer, the hydrocyclone, or the like, or it is good as needed to arrange a pump, a valve, various sensors, or the like. Incidentally, in the present description, the wording "hydrocyclone" means a separator that does not include a driving part, and that can perform separation, classification, concentration, or the like, on the liquid introduced in a static state.

The first reservoir tank 120 is a site that retains the first liquid. As shown in FIG. 1, the first reservoir tank 120 includes a connection port 121. The connection port 121 is a supply port for supplying the first liquid from the first reservoir tank 120 to a second liquid delivery line 162, and is connected to the second liquid delivery line 162. It is good that the first liquid is, for example, a washing liquid, an elution liquid, or a buffer solution, such as equilibration liquid.

Anyway, the liquid sent to the hydrocyclone 110 might contain a solid substance (for example, a carrier as described later, or the like). In a situation where, for example, an ejector is arranged on a pipe at a downstream of the hydrocyclone 110 (on the first liquid delivery line 161 in which the A liquid flows, regarding the embodiment shown in FIG. 1), there is a possibility on the above described pipe to easily cause a blockage, a retention, or the like, due to the solid substance when the hydrocyclone 110 separates the A liquid containing the solid substance from the above described liquid, so as to cause a difficulty on a flow rate control. The present inventor has studied about a configuration that suppresses the above described blockage, retention, or the like, due to the solid substance on the pipe at the downstream of the hydrocyclone 110, so as to further easily control the flow rate of the liquid on this pipe and additionally so as to efficiently merge the above described liquid with the A liquid supplied from the first reservoir tank 120. Then, as a result of an intensive study, the present inventor has reached a configuration in which the first pump 130 and the second pump 140 are provided on specific sites of the device 100.

The first pump 130 is provided at a site where the flow rate of the liquid discharged from the hydrocyclone 110 can be adjusted. In the embodiment shown by FIG. 1, the first pump 130 is provided on the first liquid delivery line 161. The first pump 130 herein is provided on the first liquid delivery line 161 at a hydrocyclone 110 side more than a confluence site joined with the second liquid delivery line 162 (a connection port 161A in FIG. 1). The first pump 130 is provided between the first derivation port 111 of the hydrocyclone 110 and the confluence site.

The second pump 140 is provided at a site where the flow rate of the first liquid discharged from the first reservoir tank 120 can be adjusted. In the embodiment shown by FIG. 1, the second pump 140 is provided on the second liquid delivery line 162. The second pump 140 herein is provided on the second liquid delivery line 162 at a first reservoir tank 120 side more than the confluence site (the connection port 161A in FIG. 1). The second pump 140 is provided between the connection port 121 of the first reservoir tank 120 and the confluence site.

In the embodiment shown by FIG. 1, the device 100 further includes an inline mixer 150. The inline mixer 150 is, for example, a mixer that mixes the liquid discharged from the hydrocyclone 110 and the first liquid discharged from the first reservoir tank 120. As shown in FIG. 1, the inline mixer 150 is arranged at the downstream of the hydrocyclone 110. The inline mixer 150 is provided on the first liquid delivery line 161 downstream of the confluence site joined with the second liquid delivery line 162 (the connection port 161A in FIG. 1). The inline mixer 150 herein includes a pipe line 151, an inflow port 152, and an outlet port 153. The pipe line 151 is formed in a tube shape (here, a circular tube shape), is a main body of the inline mixer 150, and is a flow path of the liquid. The inflow port 152 is, for example, a site where the liquid discharged from the hydrocyclone 110 flows into the pipe line 151. In the embodiment shown by FIG. 1, the inflow port 152 is provided on one end of the pipe line 151. The inflow port 152 is connected to the first liquid delivery line 161. The outlet port 153 is, for example, a site where the liquid is exhausted from the inline mixer 150. In the embodiment shown by FIG. 1, the outlet port 153 is provided at the other end of the pipe line 151. The outlet port 153 herein is connected to a pipe 172.

At a downstream of the inline mixer 150, for example, another inline mixer, another hydrocyclone, or the like, might be arranged, or a pump might be arranged as needed. Incidentally, in the present description, the wording "inline mixer" means a stationary mixer that does not include a driving part, and that uses an energy generated by a flow speed of the liquid flowing in the device so as to mix at least 2 kinds of liquids.

In the embodiment shown by FIG. 1, the device 100 includes the first liquid delivery line 161 and the second liquid delivery line 162. The first liquid delivery line 161 and the second liquid delivery line 162 herein are pipes. In this embodiment, the first liquid delivery line 161 connects the hydrocyclone 110 with the inline mixer 150. One end of the first liquid delivery line 161 is connected to the first derivation port 111 of the hydrocyclone 110. The other end of the first liquid delivery line 161 is connected to the inflow port 152 of the inline mixer 150. On the first liquid delivery line 161, the connection port 161A joined with the second liquid delivery line 162 is provided. The connection port 161A herein is a confluence site where the first liquid delivery line 161 and the second liquid delivery line 162 are joined. The second liquid delivery line 162 in this embodiment is configured to extend from the first reservoir tank 120 so as to be joined with the first liquid delivery line 161. One end of the second liquid delivery line 162 is connected to the connection port 121 of the first reservoir tank 120. The other end of the second liquid delivery line 162 is connected to the connection port 161A of the first liquid delivery line 161.

The device 100 is configured, for example, to perform an operation as described below. At first, when a switch of the supply source arranged at the upstream of the hydrocyclone 110 is turned on, the liquid is supplied from the supply source. The liquid flows from the supply source to the downstream, passes through the pipe 171, and then is introduced from the inlet port 113 into the hydrocyclone 110. Next, the liquid is separated by the hydrocyclone 110 into the A liquid and the B liquid. Here, the A liquid passes through the first derivation port 111 and then flows into the first liquid delivery line 161. The B liquid passes through the second derivation port 112 and then flows into another liquid delivery line which is different from the first liquid delivery line 161. Although not particularly restricting, it is good that said another liquid delivery line is, for example, a drainage line, a liquid delivery line directed to a purifying process for a protein, or the like. While the A liquid flows in the first liquid delivery line 161 toward the downstream (toward the confluence site joined with the second liquid delivery line 162, in the embodiment shown by FIG. 1), the flow rate of it is adjusted in response to an operation of the first pump 130. The A liquid, which flows in the first liquid delivery line 161 while receiving the operation of the first pump 130, reaches the connection port 161A.

On the other hand, from the first reservoir tank 120, the first liquid flows toward the downstream. The first liquid passes through the connection port 121 of the first reservoir tank 120 and then flows into the second liquid delivery line 162. While the first liquid flows in the second liquid delivery line 162 toward the downstream (toward the confluence site joined with the first liquid delivery line 161 in the embodiment shown by FIG. 1), the flow rate of it is adjusted in response to the operation of the second pump 140. The first liquid, which has flowed in the second liquid delivery line 162 while having received the operation of the second pump 140, reaches the connection port 161A that is the confluence site joined with the first liquid delivery line 161.

The A liquid and the first liquid are merged at the connection port 161A and further flow toward the downstream. In the embodiment shown by FIG. 1, the A liquid and the first liquid are merged at the connection port 161A and then flow toward the inline mixer 150. Then, the A liquid and the first liquid pass through the inflow port 152 and then flow into the pipe line 151 of the inline mixer 150. The A liquid and the first liquid flow toward the downstream while being mixed in the pipe line 151. A mixed liquid of the A liquid and the first liquid passes through the outlet port 153, is exhausted to an outside of the inline mixer 150, and then flows into the pipe 172. Then, the mixed liquid is subjected to processes of the pump and the hydrocyclone, which are provided at the downstream of the pipe 172, and subjected to processes of merge with another liquid, mix with this liquid, and the like.

As described above, the device 100 is a device that purifies the protein from the culture liquid of the cell. The device 100 includes the hydrocyclone 110, the first reservoir tank 120 configured to retain the first liquid, the first pump 130, and the second pump 140. The first pump 130 is provided at the site where the flow rate of the liquid discharged from the hydrocyclone 110 can be adjusted. The second pump 140 is provided at the site where the flow rate of the first liquid discharged from the first reservoir tank 120 can be adjusted.

In the device 100, the first pump 130 is provided at the site where the flow rate of the liquid discharged from the hydrocyclone 110 can be adjusted, and the second pump 140 is provided at the site where the flow rate of the first liquid discharged from the first reservoir tank 120 can be adjusted. In other words, regarding the device 100, for example, by suitably changing an output of the first pump 130, or the like, it is possible to control the flow rate of the liquid discharged from the hydrocyclone 110. Thus, even in a situation where the liquid discharged from the hydrocyclone 110 contains the solid substance, it is possible to suppress the blockage, the retention, or the like, due to the solid substance on the pipe at the downstream of the hydrocyclone 110, and therefore it is possible to further easily control the flow rate. By suitably changing the output of the second pump 140, or the like, it is possible to control the flow rate of the liquid discharged from the first reservoir tank 120. Thus, by providing the first pump 130 and the second pump 140, it is possible to further stably supply the liquid from each of the hydrocyclone 110 and the first reservoir tank 120. Therefore, the liquid discharged from the hydrocyclone 110 and the first liquid discharged from the first reservoir tank 120 can be further suitably mixed, and therefore, it is possible to further efficiently produce the purified protein from the cell culture liquid.

The device 1 might further include the inline mixer 150, the first liquid delivery line 161, and the second liquid delivery line 162. The inline mixer 150 might be arranged at the downstream of the hydrocyclone 110. The first liquid delivery line 161 might connect the hydrocyclone 110 with the inline mixer 150. The second liquid delivery line 162 might be configured to extend from the first reservoir tank 120, so as to be joined with the first liquid delivery line 161. The first pump 130 might be provided on the first liquid delivery line 161 at the hydrocyclone 110 side more than the confluence site joined with the second liquid delivery line 162 (the connection port 161A in FIG. 1). The second pump 140 might be provided on the second liquid delivery line 162 at the first reservoir tank 120 side more than the confluence site joined with the first liquid delivery line 161. In accordance with such a configuration, the first pump 130 is provided on the first liquid delivery line 161 at the hydrocyclone 110 side more than the confluence site joined with the second liquid delivery line 162, and the second pump 140 is provided on the second liquid delivery line 162 at the first reservoir tank 120 side more than the confluence site joined with the first liquid delivery line 161. In other words, the liquid discharged from the hydrocyclone 110 reaches the confluence site, after the liquid amount of it is adjusted by the first pump 130. The first liquid discharged from the first reservoir tank 120 reaches the confluence site after the liquid amount of it is adjusted by the second pump 140. Thus, both liquids are merged in states where the liquid amounts of them have been adjusted, and then are sent to the inline mixer 150. Therefore, it is possible to further enhance a mix efficiency for the liquid discharged from the hydrocyclone 110 and the first liquid discharged from the first reservoir tank 120.

The hydrocyclone 110 might be configured to separate the liquid, which has been sent to the hydrocyclone 110, into the A liquid and the B liquid. The hydrocyclone 110 might include the first derivation port 111 that derives the A liquid and include the second derivation port 112 that derives the B liquid. The first derivation port 111 might be connected to the first liquid delivery line 161. The first pump 130 might be provided between the first derivation port 111 and the above described confluence site. In accordance with such a configuration, it is possible to further enhance the mix efficiency for the liquid discharged from the hydrocyclone 110 and the first liquid discharged from the first reservoir tank 120.

It is good that the device 100 is used for purifying an antibody produced from the cultured cell. In accordance with such a configuration, it is possible to further efficiently purify the antibody from the cell culture liquid.

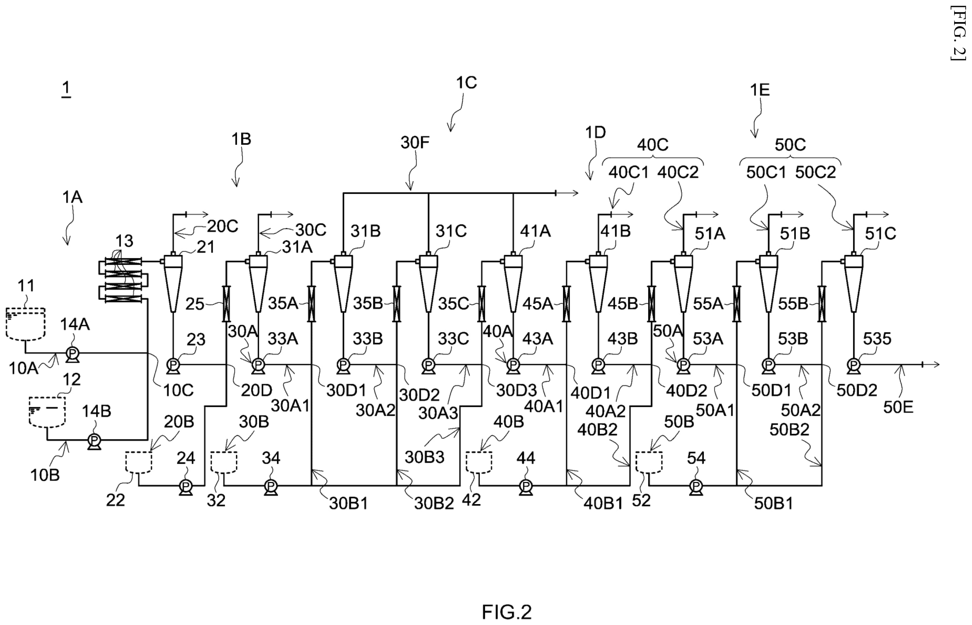

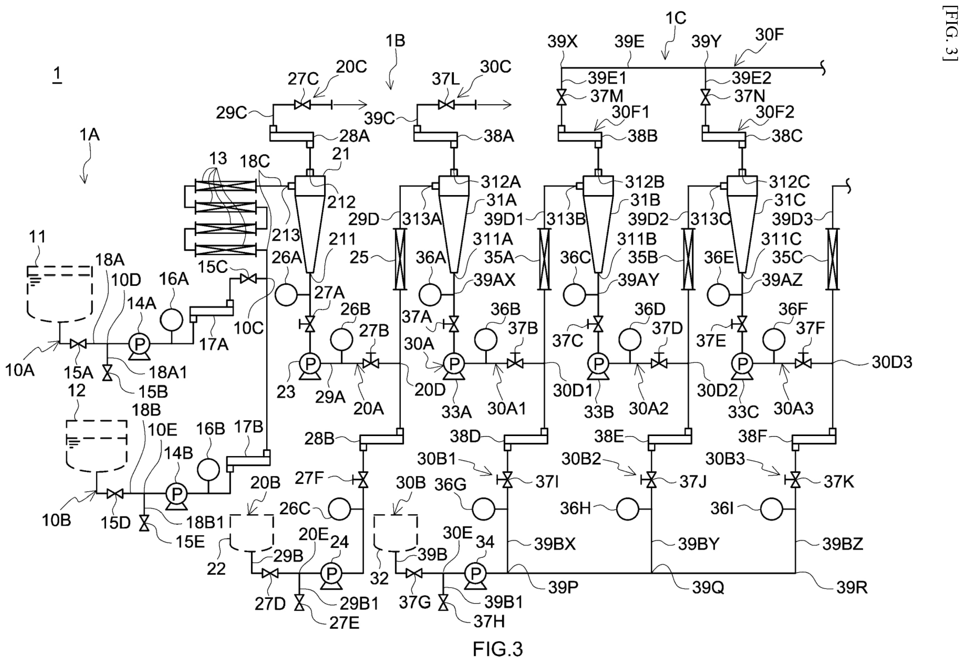

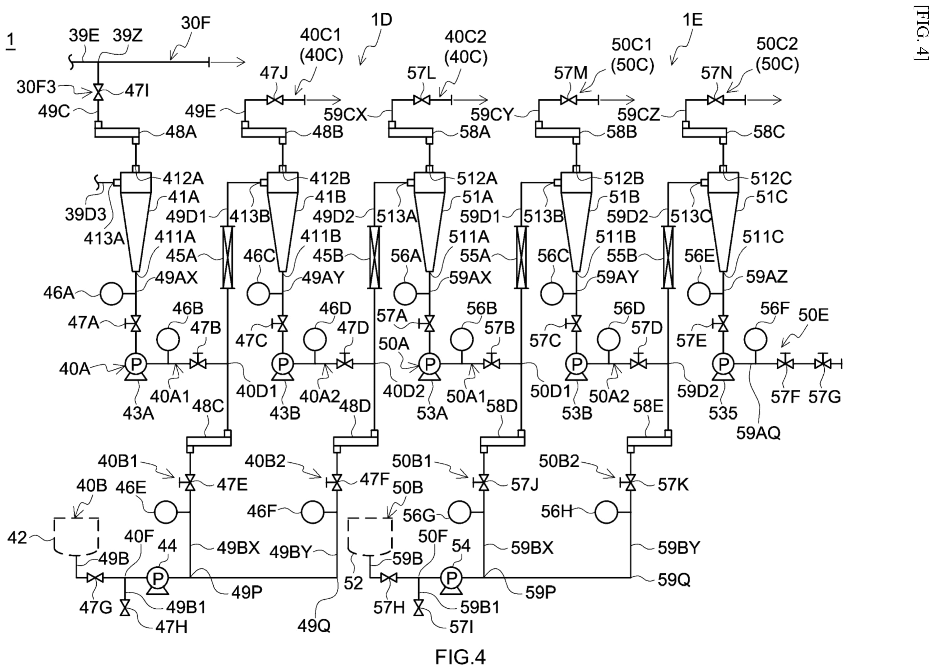

Below, while referring to FIG. 2 to FIG. 4, one embodiment of the device 100 would be explained. FIG. 2 is a schematic view of the device 1. FIG. 3 and FIG. 4 are enlarged schematic views of the device 1. FIG. 2 schematically shows a whole configuration of the device 1. FIG. 3 schematically shows configurations of a reaction module 1A, a first washing module 1B, and a part of an elution module 1C. FIG. 4 schematically shows configurations of remaining parts of the elution module 1C, a second washing module 1D, and an equilibration module 1E. The device 1 is a device that purifies the protein from the culture liquid of the cell, and includes configurations of the above described device 100. As shown in FIG. 2, the device 1 includes the reaction module 1A, the first washing module 1B, the elution module 1C, the second washing module 1D, and the equilibration module 1E. The device 1 is, for example, connected to a computer (not shown in drawings) that controls an operation of the device 1. Incidentally, in this embodiment, the liquid flows from the upstream toward the downstream.

The reaction module 1A is, for example, a module in which the culture liquid of the cell reacts with a first slurry. Regarding this embodiment, in the reaction module 1A, the culture liquid of the cell and the first slurry, which contains the carrier including a ligand binding to the protein derived from the cell, react with each other, and then a composite body in which the protein and the ligand bind to each other is generated. The culture liquid of the cell is as described above.

The first slurry herein contains the carrier that includes the ligand binding to the protein derived from the cell. The cell and the protein are as described above. It is good that the carrier including the ligand is, for example, capable of being dispersed in the liquid. Regarding the carrier including the ligand, it is preferable that the ligand and the carrier are hardly separated when the device 100 is used, or it is further preferable that both of them are not separated. As the carrier, it is possible without particular restriction to use a carrier that is used for this kind of purpose. As the carrier, for example, a bead made of resin is preferably used. The resin might be, for example, a resin made from a natural polymer chemical compound, or might be a resin made from a synthetic polymer chemical compound. It is good that the resin is, for example, a resin made from the natural polymer chemical compound, such as agarose, dextran, cellulose, chitin, chitosan, and mannan; or a resin made from the synthetic polymer chemical compound, such as polystyrene resin, and methacrylate resin. The carrier might be a bead containing an inorganic material, or might be a bead made from a glass, a silica, or a zirconia; a bead made from a stainless; or a bead made from a tungsten alloy. The carrier might be configured with a base material that is configured by mixing the above described inorganic material with the natural polymer chemical compound or the synthetic polymer chemical compound.

The ligand is to be capable of forming the composite body with the protein being the purification target. Regarding the formation of the composite body, the ligand and the protein can reversibly bind to each other. A kind of the ligand is not particularly restricted, and is suitably set in accordance with the kind of the protein being the purification target. The ligand could be, for example, an antibody binding protein, such as Protein A, Protein G, and Protein L; an antibody that recognizes the protein being the purification target (which semantically contains a primary antibody and a secondary antibody); a protein tag, such as histidine tag (His-tag), glutathione-S-transferase tag (GST tag), and FLAG (registered trademark) tag; a receptor for the protein being the purification target; or the like.

It is good that the first slurry contains a buffer solution, in addition to the carrier including the ligand that binds to the protein derived from the cell. As the buffer solution, it is possible to use a buffer solution that prevents the ligand and the protein being the purification target from being denatured. The buffer solution is preferably a buffer solution at pH 5 to pH 8, or further preferably a buffer solution at pH 6.5 to 0H 8.0. It is good that the buffer solution is, for example, a tris hydrochloride buffer solution, an acetic acid buffer solution, a citrate buffer solution, a phosphate buffer solution, or the like. As needed, a sodium chloride at 0.1 M to 1 M, a potassium chloride, or the like, might be added to the buffer solution.

As shown in FIG. 2 and FIG. 3, the reaction module 1A includes a supply source 11 for the culture liquid of the cell, a first slurry reservoir tank 12, an inline mixer 13, a hydrocyclone 21, pumps 14A, 14B, valves 15A to 15E, 27A, 27C, pressure gauges 16A, 16B, 26A, and flow meters 17A, 17B, 28A. In this embodiment, the reaction module 1A includes a culture liquid supply line 10A from the supply source 11, a first slurry supply line 10B from the first slurry reservoir tank, and a drainage line 20C. The culture liquid supply line 10A and the first slurry supply line 10B are joined at a confluence site 10C. On the confluence site 10C, a pipe 18C is arranged. To the pipe 18C, the inline mixer 13 is connected. In embodiments shown by FIG. 2 and FIG. 3, four inline mixers 13 connected in series are connected to the pipe 18C, and, at the downstream of it, the hydrocyclone 21 is further connected.

As shown in FIG. 3, from the upstream side, the culture liquid supply line 10A includes the supply source 11, the valve 15A, the valve 15B, the pump 14A, the pressure gauge 16A, the flow meter 17A, the valve 15C, the inline mixer 13, the hydrocyclone 21, the pressure gauge 26A, and the valve 27A. On the culture liquid supply line 10A, from the upstream side, the supply source 11, the valve 15A, the pump 14A, the flow meter 17A, and the valve 15C are mutually connected by a pipe 18A in this order. The valve 15B is provided on a branch pipe 18A1 that is branched from the pipe 18A at a site 10D positioned between the valve 15A and the pump 14A. The pressure gauge 16A is arranged to gauge the pressure inside the pipe 18A between the pump 14A and the flow meter 17A.

As shown in FIG. 3, the pipe 18C is connected to an inlet port 213 of the hydrocyclone 21. To a first derivation port 211 of the hydrocyclone 21, the valve 27A is connected by a pipe 29A. The pressure gauge 26A is arranged to measure the pressure inside the pipe 29A between the hydrocyclone 21 and the valve 27A.

As shown in FIG. 3, from the upstream side, the first slurry supply line 10B includes the first slurry reservoir tank 12, the valve 15D, the valve 15E, the pump 14B, the pressure gauge 16B, and the flow meter 17B. On the first slurry supply line 10B, from the upstream side, the first slurry reservoir tank 12, the valve 15D, the pump 14B, and the flow meter 17B are mutually connected by a pipe 18B in this order. The valve 15E is provided on a branch pipe 18B1 that is branched from the pipe 18B at a site 10E positioned between the valve 15D and the pump 14B. The pressure gauge 16B is arranged to gauge the pressure inside the pipe 18B between the pump 14B and the flow meter 17B.

As shown in FIG. 2 and FIG. 3, from the upstream side, the drainage line 20C includes the hydrocyclone 21, the flow meter 28A, and the valve 27C. In the embodiment shown by FIG. 3, to a second derivation port 212 of the hydrocyclone 21, a pipe 29C is connected. On the drainage line 20C, from the upstream side, the hydrocyclone 21, the flow meter 28A, and the valve 27C are mutually connected by the pipe 29C in this order. In this embodiment, the pipe 29C is connected to a drainage pathway that is not shown in drawings.

The first washing module 1B is, for example, a module that is connected to the reaction module 1A and that uses a first washing liquid so as to wash the composite body. As the first washing liquid, for example, a dispersion medium for the first slurry can be used. As shown in FIG. 2 and FIG. 3, the first washing module 1B includes a first washing liquid reservoir tank 22, a first pump 23, a second pump 24, an inline mixer 25, pressure gauges 26B, 26C, 36A, valves 27B, 27D to 27F, 37A, 37L, flow meters 28B, 38A, and a hydrocyclone 31A. The first washing module 1B includes a composite body supply line 20A that is connected to the reaction module 1A, a washing liquid supply line 20B from the first washing liquid reservoir tank 22, and a drainage line 30C. The composite body supply line 20A and the washing liquid supply line 20B are joined at a confluence site 20D.

As shown in FIG. 3, from the upstream side, the composite body supply line 20A includes the first pump 23, the pressure gauge 26B, the valve 27B, the inline mixer 25, the hydrocyclone 31A, the pressure gauge 36A, and the valve 37A. On the composite body supply line 20A, from the upstream side, the first pump 23, the valve 27B, and the inline mixer 25 are mutually connected by the pipe 29A in this order. The pressure gauge 26B is arranged to measure the pressure inside the pipe 29A between the first pump 23 and the valve 27B. The inline mixer 25 is connected to a pipe 29D at the downstream side.

As shown in FIG. 3, the pipe 29D is connected to an inlet port 313A of the hydrocyclone 31A. To a first derivation port 311A of the hydrocyclone 31A, the valve 37A is connected by a pipe 39AX. The pressure gauge 36A is arranged to measure the pressure inside the pipe 39AX between the hydrocyclone 31A and the valve 37A.

As shown in FIG. 3, from the upstream side, the washing liquid supply line 20B includes the first washing liquid reservoir tank 22, the valve 27D, the valve 27E, the second pump 24, the pressure gauge 26C, the valve 27F, and the flow meter 28B. On the washing liquid supply line 20B, from the upstream side, the first washing liquid reservoir tank 22, the valve 27D, the second pump 24, the valve 27F, and the flow meter 28B are mutually connected by a pipe 29B in this order. The valve 27E is provided on a branch pipe 29B that is branched from the pipe 29B at a site 20E positioned between the valve 27D and the second pump 24. The pressure gauge 26C is arranged to gauge the pressure inside the pipe 29B between the second pump 24 and the valve 27F.

As shown in FIG. 3, from the upstream side, the drainage line 30C includes the hydrocyclone 31A, the flow meter 38A, and the valve 37L. In this embodiment, to a second derivation port 312A of the hydrocyclone 31A, a pipe 39C is connected. On the drainage line 30C, from the upstream side, the hydrocyclone 31A, the flow meter 38A, and the valve 37L are mutually connected by the pipe 39C in this order. In this embodiment, the pipe 39C is connected to a drainage pathway that is not shown in drawings.

The elution module 1C is a module that is connected to the first washing module 1B and that uses the elution liquid so as to elute the protein from the composite body having passed through the first washing module 1B. As the elution liquid, it is possible without particular restriction to use, for example, an elution liquid that can disrupt the bind of the ligand with the protein so as to elute the protein from the composite body. The elution liquid is preferably an elution liquid at pH 5 or less, further preferably a buffer solution at pH 2 to pH 5, or furthermore preferably an elution liquid at pH 2.0 to pH 2.5. It is good that the elution liquid is, for example, an acetic acid buffer solution, a citrate buffer solution, a phosphate buffer solution, a dilute solution of phosphoric acid or hydrochloric acid, or the like. As needed, the sodium chloride, the potassium chloride, or the like, at 0.1 M to 1 M (preferably 0.1 M to 0.5 M, or further preferably 0.1 M to 0.3 M) might be added to the buffer solution.

As shown in FIG. 2 to FIG. 4, the elution module 1C includes hydrocyclones 31B, 31C, 41A, an elution liquid reservoir tank 32, first pumps 33A to 33C, a second pump 34, inline mixers 35A to 35C, pressure gauges 36B to 36I, 46A, valves 37B to 37K, 37M, 37N, 47A, 47I, and flow meters 38B to 38F, 48A. As shown in FIG. 2, the elution module 1C includes a composite body supply line 30A that is connected to the first washing module 1B and that becomes a flow path for the composite body, includes an elution liquid supply line 30B from the elution liquid reservoir tank 32, and includes a collection line 30F that collects the protein having been eluted. The composite body supply line 30A and the elution liquid supply line 30B are joined respectively at confluence sites 30D1 to 30D3.

In the embodiment shown by FIG. 2, the composite body supply line 30A includes a first block 30A1, a second block 30A2, and a third block 30A3. The first block 30A1, the second block 30A2, and the third block 30A3 are connected in series from the upstream side in this order.

As shown in FIG. 3, the first block 30A1 includes the first pump 33A, the pressure gauge 36B, the valve 37B, the inline mixer 35A, the hydrocyclone 31B, the pressure gauge 36C, and the valve 37C. In the embodiment shown by FIG. 3, on the first block 30A1, from the upstream side, the first pump 33A, the valve 37B, and the inline mixer 35A are mutually connected in this order by the pipe 39AX. The pressure gauge 36B is arranged to gauge the pressure inside the pipe 39AX between the first pump 33A and the valve 37B. In this embodiment, the inline mixer 35A and the hydrocyclone 31B are mutually connected in this order by a pipe 39D1. In this embodiment, to an inlet port 313B of the hydrocyclone 31B, the pipe 39D1 is connected. The hydrocyclone 31B and the valve 37C are mutually connected by a pipe 39AY. To a first derivation port 311B of the hydrocyclone 31B, the pipe 39AY is connected. The pressure gauge 36C is arranged to gauge the pressure inside the pipe 39AY between the hydrocyclone 31B and the valve 37C.

As shown in FIG. 3, the second block 30A2 herein includes the first pump 33B, the pressure gauge 36D, the valve 37D, the inline mixer 35B, the hydrocyclone 31C, the pressure gauge 36E, and the valve 37E. In the embodiment shown by FIG. 3, on the second block 30A2, from the upstream side, the first pump 33B, the valve 37D, and the inline mixer 35B are mutually connected in this order by the pipe 39AY. The pressure gauge 36D is arranged to gauge the pressure inside the pipe 39AY between the first pump 33B and the valve 37D . In this embodiment, the inline mixer 35B and the hydrocyclone 31C are mutually connected in this order by a pipe 39D2. In this embodiment, to an inlet port 313C of the hydrocyclone 31C, the pipe 39D2 is connected. The hydrocyclone 31C and the valve 37E are mutually connected by a pipe 39AZ. To a first derivation port 311C of the hydrocyclone 31C, the pipe 39AZ is connected. The pressure gauge 36E is arranged to gauge the pressure inside the pipe 39AZ between the hydrocyclone 31C and the valve 37E.

As shown in FIG. 3, the third block 30A3 herein includes the first pump 33C, the pressure gauge 36F, the valve 37F, the inline mixer 35C, the hydrocyclone 41A, the pressure gauge 46A, and the valve 47A. In the embodiments shown by FIG. 3 and FIG. 4, on the third block 30A3, from the upstream side, the first pump 33C, the valve 37F, and the inline mixer 35C are mutually connected in this order by the pipe 39AZ. The pressure gauge 36F is arranged to gauge the pressure inside the pipe 39AZ between the first pump 33C and the valve 37F. In this embodiment, the inline mixer 35C and the hydrocyclone 41A are mutually connected in this order by a pipe 39D3. In this embodiment, to an inlet port 413A of the hydrocyclone 41A, the pipe 39D3 is connected. The hydrocyclone 41A and the valve 47A are mutually connected by a pipe 49AX. To a first derivation port 411A of the hydrocyclone 41A, the pipe 49AX is connected. The pressure gauge 46A is arranged to gauge the pressure inside the pipe 49AX between the hydrocyclone 41A and the valve 47A.

As shown in FIG. 2 and FIG. 3, the elution liquid supply line 30B includes the elution liquid reservoir tank 32, the valve 37G, the valve 37H, the second pump 34, a first line 30B1, a second line 30B2, and a third line 30B3. In this embodiment, on the elution liquid supply line 30B, from the upstream side, the elution liquid reservoir tank 32, the valve 37G, and the second pump 34 are mutually connected in this order by a pipe 39B, and the first line 30B1, the second line 30B2, and the third line 30B3 are arranged at the downstream of the second pump 34. The valve 37H is provided on a branch pipe 39B1 that is branched from the pipe 39B at a site 30E between the valve 37G and the second pump 34.

As shown in FIG. 3, the first line 30B1 connects a branch point 39P on the pipe 39B with the confluence site 30D1. The first line 30B1 herein includes the pressure gauge 36G, the valve 37I, and the flow meter 38D. In the embodiment shown by FIG. 3, on the first line 30B1, from the branch point 39P side, the valve 37I and the flow meter 38D are mutually connected in this order by a pipe 39BX. The pressure gauge 36G is arranged to gauge the pressure inside the pipe 39BX between the branch point 39P and the valve 37I. The pipe 39BX is configured to extend with the branch point 39P being treated as a base end, and is joined to the pipe 39AX at the confluence site 30D1.

As shown in FIG. 3, the second line 30B2 connects a branch point 39Q on the pipe 39B with a confluence site 30D2. The second line 30B2 herein includes the pressure gauge 36H, the valve 37J, and the flow meter 38E. In the embodiment shown by FIG. 3, on the second line 30B2, from the branch point 39Q side, the valve 37J and the flow meter 38E are mutually connected in this order by a pipe 39BY. The pressure gauge 36H is arranged to gauge the pressure inside the pipe 39BY between the branch point 39Q and the valve 37J. The pipe 39BY is configured to extend with the branch point 39Q being treated as a base end, and is joined to the pipe 39AY at the confluence site 30D2.

As shown in FIG. 3, the third line 30B3 connects a branch point 39R on the pipe 39B with a confluence site 30D3. The third line 30B3 herein includes the pressure gauge 36I, the valve 37K, and the flow meter 38F. In the embodiment shown by FIG. 3, on the third line 30B3, from the branch point 39R side, the valve 37K and the flow meter 38F are mutually connected in this order by a pipe 39BZ. The pressure gauge 36I is arranged to gauge the pressure inside the pipe 39BZ between the branch point 39R and the valve 37K. The pipe 39BZ is configured to extend with the branch point 39R being treated as a base end, and is joined to the pipe 39AZ at the confluence site 30D3.

As shown in FIG. 3, the collection line 30F includes a first collection line 30F1, a second collection line 30F2, and a third collection line 30F3.

As shown in FIG. 3, the first collection line 30F1 connects a second derivation port 312B of the hydrocyclone 31B with a branch point 39X on a pipe 39E (a main pipe of the collection line 30F). The first collection line 30F1 herein includes the flow meter 38B and the valve 37M. In the embodiment shown by FIG. 3, on the first collection line 30F1, from the second derivation port 312B side, the flow meter 38B and the valve 37M are mutually connected in this order by a pipe 39E1. The pipe 39E1 is joined to the pipe 39E at the branch point 39X. As shown in FIG. 3, the second collection line 30F2 connects a second derivation port 312C of the hydrocyclone 31C with a branch point 39Y on the pipe 39E. The second collection line 30F2 herein includes the flow meter 38C and the valve 37N. In the embodiment shown by FIG. 3, on the second collection line 30F2, from the second derivation port 312C side, the flow meter 38C and the valve 37N are mutually connected in this order by a pipe 39E2. The pipe 39E2 is joined to the pipe 39E at the branch point 39Y. As shown in FIG. 4, the third collection line 30F3 connects a second derivation port 412A of the hydrocyclone 41A with a branch point 39Z on the pipe 39E. The third collection line 30F3 herein includes the flow meter 48A and the valve 47I. In the embodiment shown by FIG. 4, on the third collection line 30F3, from the second derivation port 412A side, the flow meter 48A and the valve 47I are mutually connected in this order by a pipe 49C. The pipe 49C is joined to the pipe 39E at the branch point 39Z.

The second washing module 1D is, for example, a module that is connected to the elution module 1C and that uses a second washing liquid so as to wash the ligand and the carrier, the ligand from which the protein has been eluted. As the second washing liquid, for example, it is possible without particular restriction to use various buffer solutions described as the first washing liquid. The second washing liquid might be the same as the first washing liquid, or might be different from the first washing liquid.

As shown in FIG. 2 and FIG. 4, the second washing module 1D includes hydrocyclones 41B, 51A, a second washing liquid reservoir tank 42, first pumps 43A, 43B, a second pump 44, inline mixers 45A, 45B, pressure gauges 46B to 46F, 56A, valves 47B to 47H, 47J, 57A, 57L, and flow meters 48B to 48D, 58A. As shown in FIG. 2, the second washing module 1D includes a carrier supply line 40A becoming a flow path for the carrier (the carrier including the ligand), includes a washing liquid supply line 40B from the second washing liquid reservoir tank 42, and includes a drainage line 40C. The carrier supply line 40A and the washing liquid supply line 40B are joined respectively at confluence sites 40D1, 40D2.

In embodiments shown by FIG. 2 and FIG. 4, the carrier supply line 40A includes a first block 40A1 and a second block 40A2. The first block 40A1 and the second block 40A2 are connected in this order from the upstream side in series. The first block 40A1 herein includes the first pump 43A, the pressure gauge 46B, the valve 47B, the inline mixer 45A, the hydrocyclone 41B, the pressure gauge 46C, and the valve 47C. In the embodiment shown by FIG. 4, on the first block 40A1, from the upstream side, the first pump 43A, the valve 47B, and the inline mixer 45A are mutually connected in this order by the pipe 49AX. The pressure gauge 46B is arranged to gauge the pressure inside the pipe 49AX between the first pump 43A and the valve 47B. In this embodiment, the inline mixer 45A and the hydrocyclone 41B are mutually connected in this order by a pipe 49D1. In this embodiment, to an inlet port 413B of the hydrocyclone 41B, the pipe 49D1 is connected. The hydrocyclone 41B and the valve 47C are mutually connected by a pipe 49AY. To a first derivation port 411B of the hydrocyclone 41B, the pipe 49AY is connected. The pressure gauge 46C is arranged to gauge the pressure inside the pipe 49AY between the hydrocyclone 41B and the valve 47C.

As shown in FIG. 4, the second block 40A2 includes the first pump 43B, the pressure gauge 46D, the valve 47D, the inline mixer 45B, the hydrocyclone 51A, the pressure gauge 56A, and the valve 57A. In the embodiment shown by FIG. 4, on the second block 40A2, from the upstream side, the first pump 43B, the valve 47D, and the inline mixer 45B are mutually connected in this order by the pipe 49AY. The pressure gauge 46D is arranged to gauge the pressure inside the pipe 49AY between the first pump 43B and the valve 47D. In this embodiment, the inline mixer 45B and the hydrocyclone 51A are mutually connected in this order by a pipe 49D2. In this embodiment, to an inlet port 513A of the hydrocyclone 51A, the pipe 49D2 is connected. The hydrocyclone 51A and the valve 57A are mutually connected by a pipe 59AX. To a first derivation port 511A of the hydrocyclone 51A, the pipe 59AX is connected. The pressure gauge 56A is arranged to gauge the pressure inside the pipe 59AX between the hydrocyclone 51A and the valve 57A.

As shown in FIG. 2 and FIG. 4, the washing liquid supply line 40B includes the second washing liquid reservoir tank 42, the valve 47G, the valve 47H, the second pump 44, a first line 40B1, and a second line 40B2. In this embodiment, on the washing liquid supply line 40B, from the upstream side, the second washing liquid reservoir tank 42, the valve 47G, and the second pump 44 are mutually connected in this order by a pipe 49B, and the first line 40B1 and the second line 40B2 are arranged at the downstream of the second pump 44. The valve 47H is provided on a branch pipe 49B1 that is branched from the pipe 49B at a site 40F between the valve 47G and the second pump 44.

As shown in FIG. 4, the first line 40B1 connects a branch point 49P on the pipe 49B with the confluence site 40D1. The first line 40B1 herein includes the pressure gauge 46E, the valve 47E, and the flow meter 48C. In the embodiment shown by FIG. 4, on the first line 40B1, from the branch point 49P side, the valve 47E and the flow meter 48C are mutually connected in this order by a pipe 49BX. The pressure gauge 46E is arranged to gauge the pressure inside the pipe 49BX between the branch point 49P and the valve 47E. The pipe 49BX is configured to extend with the branch point 49P being treated as a base end, and is joined to the pipe 49AX at the confluence site 40D1.

As shown in FIG. 4, the second line 40B2 connects a branch point 49Q on the pipe 49B with the confluence site 40D2. The second line 40B2 herein includes the pressure gauge 46F, the valve 47F, and the flow meter 48D. In the embodiment shown by FIG. 4, on the second line 40B2, from the branch point 49Q side, the valve 47F and the flow meter 48D are mutually connected in this order by a pipe 49BY. The pressure gauge 46F is arranged to gauge the pressure inside the pipe 49BY between the branch point 49Q and the valve 47F. The pipe 49BY is configured to extend with the branch point 49Q being treated as a base end, and is joined to the pipe 49AY at the confluence site 40D2.

In this embodiment, the drainage line 40C includes a first drainage line 40C1 and a second drainage line 40C2. As shown in FIG. 4, from the upstream side, the first drainage line 40C1 includes the hydrocyclone 41B, the flow meter 48B, and the valve 47J. In this embodiment, to the second derivation port 412B of the hydrocyclone 41B, a pipe 49E is connected. On the first drainage line 40C1, from the upstream side, the hydrocyclone 41B, the flow meter 48B, and the valve 47J are mutually connected in this order by the pipe 49E. In this embodiment, the pipe 49E is connected to a drainage pathway that is not shown in drawings. In the embodiment shown by FIG. 4, the second drainage line 40C2 includes the hydrocyclone 51A, the flow meter 58A, and the valve 57L. Here, to a second derivation port 512A of the hydrocyclone 51A, a pipe 59CX is connected. As shown in FIG. 4, on the second drainage line 40C2, from the upstream side, the hydrocyclone 51A, the flow meter 58A, and the valve 57L are mutually connected in this order by the pipe 59CX. In this embodiment, the pipe 59CX is connected to a drainage pathway that is not shown in drawings.

The equilibration module 1E is, for example, a module that is connected to the second washing module 1D and that uses the equilibration liquid so as to equilibrate the ligand having been washed in the second washing module 1D (the ligand provided on the carrier). In consideration that the carrier including the ligand, which has been equilibrated by this module, is returned to the first slurry reservoir tank 12, it is preferable that the equilibration liquid is the same as the dispersion medium contained in the first slurry.

As shown in FIG. 2 and FIG. 4, the equilibration module 1E includes the hydrocyclones 51B, 51C, an equilibration liquid reservoir tank 52, first pumps 53A, 53B, a second pump 54, inline mixers 55A, 55B, pressure gauges 56B to 56E, 56G, 56H, valves 57B to 57E, 57H to 57K, 57M, 57N, and flow meters 58B to 58E. As shown in FIG. 2, the equilibration module 1E includes a carrier supply line 50A that becomes a flow path for the carrier (the carrier including the ligand), an equilibration liquid supply line 50B from the equilibration liquid reservoir tank 52, and a drainage line 50C. The carrier supply line 50A and the equilibration liquid supply line 50B are joined respectively at confluence sites 50D1, 50D2.

In embodiments shown by FIG. 2 and FIG. 4, the carrier supply line 50A includes a first block 50A1 and a second block 50A2. The first block 50A1 and the second block 50A2 are connected in this order from the upstream side in series. As shown in FIG. 4, the first block 50A1 herein includes the first pump 53A, the pressure gauge 56B, the valve 57B, the inline mixer 55A, the hydrocyclone 51B, the pressure gauge 56C, and the valve 57C. In the embodiment shown by FIG. 4, on the first block 50A1, from the upstream side, the first pump 53A, the valve 57B, and the inline mixer 55A are mutually connected in this order by the pipe 59AX. The pressure gauge 56B is arranged to gauge the pressure inside the pipe 59AX between the first pump 53A and the valve 57B. In this embodiment, the inline mixer 55A and the hydrocyclone 51B are mutually connected in this order by a pipe 59D1. In this embodiment, to an inlet port 513B of the hydrocyclone 51B, the pipe 59D1 is connected. The hydrocyclone 51B and the valve 57C are mutually connected by a pipe 59AY. To a first derivation port 511B of the hydrocyclone 51B, the pipe 59AY is connected. The pressure gauge 56C is arranged to gauge the pressure inside the pipe 59AY between the hydrocyclone 51B and the valve 57C.

As shown in FIG. 4, the second block 50A2 includes the first pump 53B, the pressure gauge 56D, the valve 57D, the inline mixer 55B, the hydrocyclone 51C, the pressure gauge 56E, and the valve 57E. In the embodiment shown by FIG. 4, on the second block 50A2, from the upstream side, the first pump 53B, the valve 57D, and the inline mixer 55B are mutually connected in this order by the pipe 59AY. The pressure gauge 56D is arranged to gauge the pressure inside the pipe 59AY between the first pump 53B and the valve 57D. In this embodiment, the inline mixer 55B and the hydrocyclone 51C are mutually connected in this order by a pipe 59D2. In this embodiment, to an inlet port 513C of the hydrocyclone 51C, the pipe 59D2 is connected. The hydrocyclone 51C and the valve 57E are mutually connected by a pipe 59AZ. To a first derivation port 511C of the hydrocyclone 51C, the pipe 59AZ is connected. The pressure gauge 56E is arranged to gauge the pressure inside the pipe 59AZ between the hydrocyclone 51C and the valve 57E.

As shown in FIG. 4, the equilibration liquid supply line 50B includes the equilibration liquid reservoir tank 52, the valve 57H, the valve 57I, the second pump 54, a first line 50B1, and a second line 50B2. In this embodiment, on the equilibration liquid supply line 50B, from the upstream side, the equilibration liquid reservoir tank 52, the valve 57H, and the second pump 54 are mutually connected in this order by a pipe 59B, and the first line 50B1 and the second line 50B2 are arranged at the downstream of the second pump 54. The valve 57I is provided on a branch pipe 59B 1 that is branched from the pipe 59B at a site 50F between the valve 57H and the second pump 54.

As shown in FIG. 4, the first line 50B1 connects a branch point 59P on the pipe 59B with the confluence site 50D1. The first line 50B1 herein includes the pressure gauge 56G, the valve 57J, and the flow meter 58D. In the embodiment shown by FIG. 4, on the first line 50B1, from the branch point 59P side, the valve 57J and the flow meter 58D are mutually connected in this order by a pipe 59BX. The pressure gauge 56G is arranged to gauge the pressure inside the pipe 59BX between the branch point 59P and the valve 57J. The pipe 59BX is configured to extend with the branch point 59P being treated as a base end, and is joined to the pipe 59AX at the confluence site 50D1.

As shown in FIG. 4, the second line 50B2 connects a branch point 59Q on the pipe 59B with the confluence site 50D2. The second line 50B2 herein includes the pressure gauge 56H, the valve 57K, and the flow meter 58E. In the embodiment shown by FIG. 4, on the second line 50B2, from the branch point 59Q side, the valve 57K and the flow meter 58E are mutually connected in this order by a pipe 59BY. The pressure gauge 56H is arranged to gauge the pressure inside the pipe 59BY between the branch point 59Q and the valve 57K. The pipe 59BY is configured to extend with the branch point 59Q being treated as a base end, and is joined to the pipe 59AY at the confluence site 50D2.

As shown in FIG. 2, the drainage line 50C includes a first drainage line 50C1 and a second drainage line 50C2. The first drainage line 50C1 includes the hydrocyclone 51B, the flow meter 58B, and the valve 57M. Here, to a second derivation port 512B of the hydrocyclone 51B, a pipe 59CY is connected. As shown in FIG. 4, from the upstream side, the hydrocyclone 51B, the flow meter 58B, and the valve 57M are mutually connected in this order by the pipe 59CY. In this embodiment, the pipe 59CY is connected to a drainage pathway that is not shown in drawings. The second drainage line 50C2 includes the hydrocyclone 51C, the flow meter 58C, and the valve 57N. Here, to a second derivation port 512C of the hydrocyclone 51C, a pipe 59CZ is connected. As shown in FIG. 4, on the second drainage line 50C2, from the upstream side, the hydrocyclone 51C, the flow meter 58C, and the valve 57N are mutually connected in this order by the pipe 59CZ. In this embodiment, the pipe 59CZ is connected to a drainage pathway that is not shown in drawings.

In this embodiment, to the equilibration module 1E, a return line 50E is connected. The return line 50E herein is a line that returns the carrier, which has been equilibrated, to the first slurry reservoir tank 12. In the embodiment shown by FIG. 4, to the downstream of the valve 57E of the equilibration module 1E, a pipe 59AQ (a main pipe of the return line 50E in this embodiment) is connected. As shown in FIG. 4, the return line 50E includes a pump 535, a pressure gauge 56F, a valve 57F, and a valve 57G. In the embodiments shown by FIG. 2 and FIG. 4, on the return line 50E, from the upstream side, the pump 535, the valve 57F, and the valve 57G are mutually connected in this order by the pipe 59AQ. The pressure gauge 56F is arranged to gauge the pressure inside the pipe 59AQ between the pump 535 and the valve 57F.

Next, while referring to FIG. 2 to FIG. 4, an operation of the device 1 would be explained. However, the explanation described below is to explain an example of the operation of the device 1, and is not intended to restrict the herein disclosed technique. When a switch of the computer controlling the operation of the device 1 is turned on and then a switch of the device 1 is turned on, the device 1 becomes operable. At first, on the reaction module 1A, the first slurry is sent from the first slurry reservoir tank 12 to the first slurry supply line 10B. At that time, it is good that the valve 15D is in an open state and that the pump 14B is in an operable state. It is good that the valve 15E is in a close state. The first slurry passes from the first slurry reservoir tank 12 through the pipe 18B, and reaches to the confluence site 10C while a liquid amount inside the pipe 18B is adjusted by the pump 14B. An output of the pump 14B can be suitably set, for example, according to measurement values of the pressure gauge 16B and the flow meter 17B which are provided on the first slurry supply line 10B.

As described above, while the first slurry is in a state of being sent, the culture liquid of the cell is sent from the supply source 11 to the culture liquid supply line 10A. At that time, it is good that the valve 15A and the valve 15C are in open states and that the pump 14A is in an operable state. It is good that the valve 15B is in a close state. The culture liquid passes from the supply source 11 through the pipe 18A, and reaches to the confluence site 10C while a liquid amount of it inside the pipe 18A is adjusted by the pump 14A. Incidentally, an output of the pump 14A can be suitably set, for example, according to measurement values of the pressure gauge 16A and the flow meter 17A which are provided on the culture liquid supply line 10A.

The culture liquid and the first slurry, which have been merged at the confluence site 10C, pass through the pipe 18C and are mixed by the four inline mixers 13 which are connected in series. At that time, the protein in the culture liquid binds with the ligand in the first slurry, and then the composite body of the protein with the ligand is generated in a mixed liquid of the culture liquid and the first slurry (below, which might be referred to as "first mixed liquid", too). Then, the first mixed liquid passes through the inline mixer 13 and then is sent to the hydrocyclone 21.

The first mixed liquid passes from the pipe 18C through the inlet port 213 and then enters into the hydrocyclone 21. By the hydrocyclone 21, the first mixed liquid is separated into a component A and a component B. The component A contains, for example, both of a carrier including the composite body and a carrier including a ligand that has not been reacted yet. The component B is, for example, a remaining component. The component A is sent from the first derivation port 211 of the hydrocyclone 21 to the pipe 29A, passes through a site of the valve 27A, and then enters into the first washing module 1B. At that time, it is good that the valve 27A and the valve 27B are in open states and that the first pump 23 is in an operable state. The component A passes from the first derivation port 211 through the pipe 29A, and reaches to the confluence site 20D while a liquid amount of it inside the pipe 29A is adjusted by the first pump 23. Incidentally, an output of the first pump 23 can be suitably set, for example, according to measurement values of the pressure gauge 26A and the pressure gauge 26B.

The component B is discharged by the drainage line 20C to the drainage pathway. The component B herein passes from the second derivation port 212 of the hydrocyclone 21 through the pipe 29C, and then is discharged to the drainage pathway. At that time, it is good that the valve 27C is in an open state.

At that moment, on the washing liquid supply line 20B, the first washing liquid is sent from the first washing liquid reservoir tank 22 to the pipe 29B. At that time, it is good that the valve 27D is in an open state and that the second pump 24 is in an operable state. It is good that the valve 27E is in a close state. The first washing liquid passes from the first washing liquid reservoir tank 22 through the pipe 29B, reaches to the confluence site 20D while a liquid amount of it inside the pipe 29B is adjusted by the second pump 24, and then is merged into the composite body supply line 20A. Incidentally, an output of the second pump 24 can be suitably set, for example, by measurement values of the pressure gauge 26C and the flow meter 28B.

The component A and the first washing liquid, which have been merged at the confluence site 20D, pass through the pipe 29A, and then are mixed by the inline mixer 25. At that time, for example, the composite body is washed with the first washing liquid, and thus components non-specifically adhering to the carrier, the composite body, or the like (for example, proteins derived from the culture liquid, or the like) are removed, and float in the liquid. Then, a mixed liquid of the component A with the first washing liquid (below, which might be simply referred to as "second mixed liquid", too) passes through the inline mixer 25, and then is sent to the hydrocyclone 31A.

Then, the second mixed liquid passes from the pipe 29D through the inlet port 313A, and enters into the hydrocyclone 31A. By the hydrocyclone 31A, the second mixed liquid is separated into a component C and a component D. The component C contains, for example, the carrier including the composite body and the carrier including a ligand that has not been reacted yet. The component C is sent from the first derivation port 311A of the hydrocyclone 31A to the pipe 39AX, passes through a site of the valve 37A, and then enters into the first block 30A1 of the composite body supply line 30A on the elution module 1C. At that time, it is good that the valve 37A and the valve 37B are in open states, and that the first pump 33A is in an operable state. The component C passes from the first derivation port 311A through the pipe 39AX, and reaches to the confluence site 30D1 while a liquid amount of it inside the pipe 39AX is adjusted by the first pump 33A. Incidentally, an output of the first pump 33A can be suitably set, for example, according to measurement values of the pressure gauge 36A and the pressure gauge 36B. The component D is discharged to the drainage pathway by the drainage line 30C. The component D herein passes from the second derivation port 312A of the hydrocyclone 31A through the pipe 39C, and then is discharged to the drainage pathway. At that time, it is good that the valve 37L is in an open state.

On the other hand, on the elution liquid supply line 30B, the elution liquid is sent from the elution liquid reservoir tank 32 to the pipe 39B. At that time, it is good that the valve 37G is in an open state and that the second pump 34 is in an operable state. It is good that the valve 37H is in a close state. Further, it is good that the valve 37I on the pipe 39BX is in an open state. The elution liquid passes from the elution liquid reservoir tank 32 through the pipe 39B and the pipe 39BX, reaches to the confluence site 30D1 while a liquid amount inside the pipe 39B and a liquid amount inside the pipe 39BX are adjusted by the second pump 34, and then is merged into the first block 30A1 of the composite body supply line 30A. Incidentally, an output of the second pump 34 can be suitably set, for example, according to measurement values of the pressure gauge 36G and the flow meter 38D provided on the pipe 39BX, measurement values of the pressure gauge 36H and the flow meter 38E provided on the pipe 39BY described later, and measurement values of the pressure gauge 36I and the flow meter 38F provided on the pipe 39BZ described later.

The component C and the elution liquid, which have been merged at the confluence site 30D1, pass through the pipe 39AX, and then are mixed by the inline mixer 35A. At that time, for example, by the elution liquid, the protein is eluted from the composite body into the liquid. Then, a mixed liquid of the component C and the elution liquid (below, which might be simply referred to as "third mixed liquid", too) passes through the inline mixer 35A, and then sent to the hydrocyclone 31B.

Then, the third mixed liquid passes from the pipe 39D1 through the inlet port 313B, and enters into the hydrocyclone 31B. By the hydrocyclone 31B, the third mixed liquid is separated into a component E and a component F. The component E can contain a carrier including the ligand from which the protein has been eluted on the first block 30A1, and contain a carrier including the composite body from which the protein has not been eluted yet on the first block 30A1. The component F is a remaining component, and can contain the protein eluted from the first block 30A1. The component E is sent from the first derivation port 311B of the hydrocyclone 31B to the pipe 39AY, passes through a site of the valve 37C, and then enters into the second block 30A2. At that time, it is good that the valve 37C and the valve 37D are in open states, and that the first pump 33B is in an operable state. The component E passes from the first derivation port 311B through the pipe 39AY, and reaches to the confluence site 30D2 while a liquid amount of it inside the pipe 39AY is adjusted by the first pump 33B. Incidentally, an output of the first pump 33B can be suitably set, for example, according to measurement values of the pressure gauge 36C and the pressure gauge 36D.

Incidentally, the component F is sent to the collection line 30F by the first collection line 30F1. The component F passes from the second derivation port 312B of the hydrocyclone 31B through the pipe 39E1, and then is sent from the branch point 39X to the pipe 39E. At that time, it is good that the valve 37M is in an open state. After that, the component F is merged with a component H and a component J at the pipe 39E, which are described later, and then sent to a protein purifying process.

On the other hand, on the elution liquid supply line 30B, the valve 37G is in an open state, the second pump 34 is in an operable state, the elution liquid is sent from the elution liquid reservoir tank 32 to the pipe 39B, and then, by making the valve 37J be in an open state on the pipe 39BY, the second line 30B2 is opened. The elution liquid passes from the elution liquid reservoir tank 32 through the pipe 39B and the pipe 39BY, reaches to the confluence site 30D2 while a liquid amount inside the pipe 39B and a liquid amount inside the pipe 39BY are adjusted by the second pump 34, and then is merged into the second block 30A2 of the composite body supply line 30A.

The component E and the elution liquid, which have been merged at the confluence site 30D2, pass through the pipe 39AY, and then are mixed by the inline mixer 35B. At that time, for example, with the elution liquid, the protein is eluted from the composite body in the liquid. Then, a mixed liquid of the component E and the elution liquid (below, which might be simply referred to as "fourth mixed liquid", too) passes through the inline mixer 35B, and then is sent to the hydrocyclone 31C.

Next, the fourth mixed liquid passes from the pipe 39D2 through the inlet port 313C, and then enters into the hydrocyclone 31C. By the hydrocyclone 31C, the fourth mixed liquid is separated into a component G and a component H. The component G can contain a carrier including a ligand from which the protein has been eluted on the first block 30A1 and the second block 30A2, and contain a carrier including the composite body from which the protein has not been eluted on the second block 30A2. The component H is a remaining component, and can contain the protein having been eluted on the second block 30A2. The component G is sent from the first derivation port 311C of the hydrocyclone 31C to the pipe 39AZ, passes through a site of the valve 37E, and then enters into the third block 30A3. At that time, it is good that the valve 37E and the valve 37F are in open states, and that the first pump 33C is in an operable state. The component G passes from the first derivation port 311C through the pipe 39AZ, and reaches to the confluence site 30D3 while a liquid amount of it inside the pipe 39AZ is adjusted by the first pump 33C. Incidentally, an output of the first pump 33C can be suitably set, for example, according to measurement values of the pressure gauge 36E and the pressure gauge 36F.

Incidentally, the component H is sent to the collection line 30F by the second collection line 30F2. The component H passes from the second derivation port 312C of the hydrocyclone 31C through the pipe 39E2, and then is sent from the branch point 39Y to the pipe 39E. At that time, it is good that the valve 37N is in an open state. After that, the component H is merged with the component F and a later described component J at the pipe 39E, and then sent to the protein purifying process.

On the other hand, on the elution liquid supply line 30B, as described above, while the elution liquid is sent from the elution liquid reservoir tank 32 to the pipe 39B, the third line 30B3 is opened by making the valve 37K on the pipe 39BZ be in the open state. The elution liquid passes from the elution liquid reservoir tank 32 through the pipe 39B and the pipe 39BZ, reaches to the confluence site 30D3 while a liquid amount inside the pipe 39B and a liquid amount inside the pipe 39BZ are adjusted by the second pump 34, and then is merged at the third block 30A3 of the composite body supply line 30A.

The component G and the elution liquid, which have been merged at the confluence site 30D3, pass through the pipe 39AZ and then are mixed by the inline mixer 35C. At that time, for example, with the elution liquid, the protein is eluted from the composite body into the liquid. Then, a mixed liquid of the component G and the elution liquid (below, which is simply referred to as "fifth mixed liquid", too) passes through the inline mixer 35C, and then is sent to the hydrocyclone 41A.

The fifth mixed liquid passes from the pipe 39D3 through the inlet port 413A, and then enters into the hydrocyclone 41A. By the hydrocyclone 41A, the fifth mixed liquid is separated into a component I and a component J. The component I can contain a carrier including the ligand from which the protein has been eluted on the first block 30A1, the second block 30A2, and the third block 30A3. The component J is a remaining component, and can contain the protein that has been eluted on the third block 30A3. The component I is sent from the first derivation port 411A of the hydrocyclone 41A to the pipe 49AX, passes through a site of the valve 47A, and then enters into the first block 40A1 on the carrier supply line 40A of the second washing module 1D. At that time, it is good that the valve 47A and the valve 47B are in open states and that the first pump 43A is in an operable state. The component I passes from the first derivation port 411A through the pipe 49AX, and reaches to the confluence site 40D1 while a liquid amount of it inside the pipe 49AX is adjusted by the first pump 43A. Incidentally, an output of the first pump 43A can be suitably set, for example, according to measurement values of the pressure gauge 46A and the pressure gauge 46B.

Incidentally, the component J is sent by the third collection line 30F3 to the collection line 30F. The component J passes from the second derivation port 412A of the hydrocyclone 41A through the pipe 49C, and then is sent from the branch point 39Z to the pipe 39E. At that time, it is good that the valve 47I is in an open state. After that, the component J is merged with the component F and the component H at the pipe 39E, which are described above, and then sent to the protein purifying process.

On the other hand, on the washing liquid supply line 40B, the second washing liquid is sent from the second washing liquid reservoir tank 42 to the pipe 49B. At that time, it is good that the valve 47G is in an open state and that the second pump 44 is in an operable state. It is good that the valve 47H is in a close state. Further, it is good that the valve 47E on the pipe 49BX is in an open state. The second washing liquid passes from the second washing liquid reservoir tank 42 through the pipe 49B and the pipe 49BX, reaches to the confluence site 40D1 while a liquid amount inside the pipe 49B and a liquid amount inside the pipe 49BX are adjusted by the second pump 44, and then is merged on the first block 40A1 of the carrier supply line 40A. Incidentally, an output of the second pump 44 can be suitably set, for example, according to measurement values of the pressure gauge 46E and the flow meter 48C, which are provided on the pipe 49BX, and according to measurement values of the pressure gauge 46F and the flow meter 48D, which are described later and provided on the later pipe 49BY.

The component I and the second washing liquid, which have been merged at the confluence site 40D1, pass through the pipe 49AX and then are mixed by the inline mixer 45A. At that time, for example, with the second washing liquid, the carrier is washed. Then, a mixed liquid of the component I and the second washing liquid (below, which is simply referred to as "sixth mixed liquid", too) passes through the inline mixer 45A and is sent to the hydrocyclone 41B.

Next, the sixth mixed liquid enters into the hydrocyclone 41B. By the hydrocyclone 41B, the sixth mixed liquid is separated into a component K and a component L. The component K can contain the carrier which has been washed on the first block 40A1. The component L is a remaining component. The component K is sent from the first derivation port 411B of the hydrocyclone 41B to the pipe 49AY, passes through a site of the valve 47C, and then enters into the second block 40A2. At that time, it is good that the valve 47C and the valve 47D are in open states and that the first pump 43B is in an operable state. The component K passes from the first derivation port 411B through the pipe 49AY, and reaches to the confluence site 40D2 while a liquid amount of it inside the pipe 49AY is adjusted by the first pump 43B. Incidentally, an output of the first pump 43B can be suitably set, for example, according to measurement values of the pressure gauge 46C and the pressure gauge 46D.

Incidentally, the component L is discharged to the drainage pathway by the first drainage line 40C1. The component L herein passes from the second derivation port 412B of the hydrocyclone 41B through the pipe 49E, and then is discharged to the drainage pathway. At that time, it is good that the valve 47J is in an open state.

On the other hand, on the washing liquid supply line 40B, the valve 47G is in an open state, the second pump 44 is in an operable state, the second washing liquid is sent from the second washing liquid reservoir tank 42 to the pipe 49B, and thus, the second line 40B2 is opened by making the valve 47F on the pipe 49BY be in the open state. The second washing liquid passes from the second washing liquid reservoir tank 42 through the pipe 49B and the pipe 49BY, reaches to the confluence site 40D2 while a liquid amount inside the pipe 49B and a liquid amount inside the pipe 49BY are adjusted by the second pump 44, and then is merged on the second block 40A2 of the carrier supply line 40A.

The component K and the second washing liquid, which have been merged at the confluence site 40D2, pass through the pipe 49AY and then are mixed by the inline mixer 45B. At that time, for example, with the second washing liquid, the carrier is washed. Then, a mixed liquid of the component K and the second washing liquid (below, which is simply referred to as "seventh mixed liquid", too) passes through the inline mixer 45B, and then is sent to the hydrocyclone 51A.

The seventh mixed liquid passes from the pipe 49D2 through the inlet port 513A, and then enters into the hydrocyclone 51A. By the hydrocyclone 51A, the seventh mixed liquid is separated into a component M and a component N. The component M can contain the carrier that has been washed on the second block 40A2. The component N is a remaining component. The component M is sent from the first derivation port 511A of the hydrocyclone 51A to the pipe 59AX, passes through a site of the valve 57A, and then enters into the first block 50A1 of the carrier supply line 50A of the equilibration module 1E. At that time, it is good that the valve 57A and the valve 57B are in open states and that the first pump 53A is in an operable state. The component M passes from the first derivation port 511A through the pipe 59AX, and reaches to the confluence site 50D1 while a liquid amount of it inside the pipe 59AX is adjusted by the first pump 53A. Incidentally, an output of the first pump 53A can be suitably set, for example, according to measurement values of the pressure gauge 56A and the pressure gauge 56B.

The component N is discharged to the drainage pathway by the second drainage line 40C2. The component N herein passes from the second derivation port 512A of the hydrocyclone 51A through the pipe 59CX, and then is discharged to the drainage pathway. At that time, it is good that the valve 57L is in an open state.

On the other hand, on the equilibration liquid supply line 50B, the equilibration liquid is sent from the equilibration liquid reservoir tank 52 to the pipe 59B. At that time, it is good that the valve 57H is in an open state and that the second pump 54 is in an operable state. It is good that the valve 57I is in a close state. Further, it is good that the valve 57J on the pipe 59BX is in an open state. The equilibration liquid passes from the equilibration liquid reservoir tank 52 through the pipe 59B and the pipe 59BX, reaches to the confluence site 50D1 while a liquid amount inside the pipe 59B and a liquid amount inside the pipe 59BX are adjusted by the second pump 54, and then is merged on the first block 50A1 of the carrier supply line 50A. Incidentally, an output of the second pump 54 can be suitably set, for example, according to measurement values of the pressure gauge 56G and the flow meter 58D, which are provided on the pipe 59BX, and according to measurement values of the pressure gauge 56H and the flow meter 58E, which are provided on the pipe 59BY and described later.

The component M and the equilibration liquid, which have been merged at the confluence site 50D1, pass through the pipe 59AX, and then are mixed by the inline mixer 55A. At that time, for example, with the equilibration liquid, the carrier is equilibrated. Then, a mixed liquid of the component M and the equilibration liquid (below, which is simply referred to as "eighth mixed liquid") passes through the inline mixer 55A, and then is sent to the hydrocyclone 51B.

Next, the eighth mixed liquid passes from the pipe 59D1 through the inlet port 513B, and then enters into the hydrocyclone 51B. The eighth mixed liquid is separated into a component O and a component P by the hydrocyclone 51B. The component O can contain the carrier including the ligand that has been equilibrated on the first block 50A1. The component P is a remaining component. The component O is sent from the first derivation port 511B of the hydrocyclone 51B to the pipe 59AY, passes through a site of the valve 57C, and then enters into the second block 50A2 on the carrier supply line 50A. At that time, it is good that the valve 57C and the valve 57D are in open states and that the first pump 53B is in an operable state. The component O passes from the first derivation port 511B through the pipe 59AY, and then reaches to the confluence site 50D2 while a liquid amount of it inside the pipe 59AY is adjusted by the first pump 53B. Incidentally, an output of the first pump 53B can be suitably set, for example, according to measurement values of the pressure gauge 56C and the pressure gauge 56D.

The component P is discharged to the drainage pathway by the first drainage line 50C1. The component P herein passes from the second derivation port 512B of the hydrocyclone 51B through the pipe 59CY, and then is discharged to the drainage pathway. At that time, it is good that the valve 57M is in an open state.

On the other hand, on the equilibration liquid supply line 50B, the valve 57H is in an open state, the second pump 54 is in an operable state, the equilibration liquid is sent from the equilibration liquid reservoir tank 52 to the pipe 59B, and thus the second line 50B2 is opened by making the valve 57K on the pipe 59BY be in a open state. The equilibration liquid passes from the equilibration liquid reservoir tank 52 through the pipe 59B and the pipe 59BY, reaches to the confluence site 50D2 while a liquid amount inside the pipe 59B and a liquid amount inside the pipe 59BY are adjusted by the second pump 54, and then is merged on the second block 50A2 of the carrier supply line 50A.

The component O and the equilibration liquid, which have been merged at the confluence site 50D2, pass through the pipe 59AY and then are mixed by the inline mixer 55B. At that time, for example, the carrier onto which a process with the equilibration liquid has been performed at the first block 50A1 is further subjected to the process with the equilibration liquid. Then, a mixed liquid of the component O and the equilibration liquid (below, which is simply referred to as "ninth mixed liquid", too) passes through the inline mixer 55B and then is sent to the hydrocyclone 51C.

Next, the ninth mixed liquid passes from the pipe 59D2 through the inlet port 513C, and then enters into the hydrocyclone 51C. The ninth mixed liquid is separated into a component Q and a component R by the hydrocyclone 51C. The component Q can contain the carrier including the ligand that has been equilibrated by the second block 50A2. The component R is a remaining component. The component Q is sent from the first derivation port 511C of the hydrocyclone 51C to the pipe 59AZ, passes through the valve 57E, and then enters into the return line 50E. At that time, it is good that the valve 57E and the valve 57F are in open states and that the pump 535 is in an operable state. The component Q passes from the first derivation port 511C through the pipe 59AZ, and enters into the return line 50E, while it enters into the pipe AQ and a liquid amount of it inside the pipe 59AQ is adjusted by the pump 535. The component Q having entered into the return line 50E is finally sent to the first slurry reservoir tank 12 of the reaction module 1A. Incidentally, an output of the pump 535 can be suitably set, for example, according to measurement values of the pressure gauge 56E and the pressure gauge 56F.

The component R is discharged to the drainage pathway by the second drainage line 50C2. The component R herein passes from the second derivation port 512C of the hydrocyclone 51C through the pipe 59CZ, and then is discharged to the drainage pathway. At that time, it is good that the valve 57N is in an open state.

As described above, the configuration of the device 100 shown in FIG. 1 can be included in the device 1 shown by FIG. 2 to FIG. 4. The device 100 might include configurations of the hydrocyclone 110, the first reservoir tank 120, the first pump 130, and the second pump 140 which are shown in FIG. 1 (below, which are simply referred to as "configurations shown in FIG. 1", too). The hydrocyclones 21, 31A to 31C, 41A, 41B, 51A, 51B of the device 1 shown in FIG. 2 to FIG. 4 can correspond to the hydrocyclone 110 shown on FIG. 1. The first washing liquid reservoir tank 22 of the device 1, the elution liquid reservoir tank 32, the second washing liquid reservoir tank 42, and the equilibration liquid reservoir tank 52 can correspond to the first reservoir tank 120 of FIG. 1. The first pumps 23, 33A to 33C, 43A, 43B, 53A, 53B of the device 1 can correspond to the first pump 130 of FIG. 1. The second pumps 24, 34, 44, 54 of the device 1 can correspond to the second pump 140 of FIG. 1. By making the device 1 include the configurations shown in FIG. 1, it is possible to further efficiently produce the purified protein from the cell culture liquid.

In this embodiment, regarding the device 1, in order to capture the protein being the purification target, a carrier capable of being dispersed in the liquid is used as the carrier including the ligand for this protein. For this matter, in the device 1, the first pumps 23, 33A to 33C, 43A, 43B, 53A, 53B are arranged at the downstream of the hydrocyclones 21, 31A to 31C, 41A, 41B, 51A, 51B. By the first pumps 23, 33A to 33C, 43A, 43B, 53A, 53B, it is possible to suppress the blockage, the retention, or the like, in the pipe due to the carrier of the liquid containing the carriers that have been discharged from the hydrocyclones 21, 31A to 31C, 41A, 41B, 51A, 51B respectively positioned at the upstream of the first pumps, and consequently, it becomes easier to control the flow rate of the liquid. Thus, a function of each module can be implemented further properly.

Although not particularly restricting, it is preferable that the configuration shown in FIG. 1 is provided on the elution module 1C. By providing the configuration shown in FIG. 1 on the elution module 1C, the liquids discharged from the hydrocyclones 31B, 31C, 41A and the elution liquid discharged from the elution liquid reservoir tank 32 can be mixed further properly, thus the protein is further efficiently eluted from the composite body, and consequently, it is possible to further efficiently produce the purified protein.

The protein being the purification target might be an antibody. The ligand might be an antibody binding protein. In accordance with such a configuration, it is possible to further efficiently purify the antibody from the cell culture liquid.

According to the herein disclosed technique, the method for producing the purified protein is provided that uses the device 1 or the device 100 and that includes purifying the protein from the culture liquid of the cell. By using the producing method described above, it is possible to further efficiently produce the purified protein from the cell culture liquid.

Incidentally, as described above, it is enough for the device 1 to include the configuration shown in FIG. 1, and thus a number of the hydrocyclones, a number of the inline mixers, a number of the first pumps, a number of the second pumps, or the like, in each module is not particularly restricted. The numbers as described above can be suitably set according to a scale of the device, an amount of raw materials processed in one operation of the device, or the like.

The herein disclosed technique can contain an embodiment recited in each item described below.
-Item 1-A device that purifies a protein from a culture liquid of a cell, comprising:
   a hydrocyclone;
   a first reservoir tank that retains a first liquid;
   a first pump; and
   a second pump, wherein
   the first pump is provided at a site where a flow rate of a liquid discharged from the hydrocyclone can be adjusted, and
   the second pump is provided at a site where a flow rate of the first liquid discharged from the first reservoir tank can be adjusted.
-Item 2-The device recited in Item 1, further comprising:
   an inline mixer that is positioned at a downstream of the hydrocyclone;
   a first liquid delivery line that connects the hydrocyclone and the inline mixer; and
   a second liquid delivery line that extends from the first reservoir tank and that is joined to the first liquid delivery line, wherein
   on the first liquid delivery line, the first pump is provided at a position closer to the hydrocyclone than to a confluence site joined with the second liquid delivery line, and
   on the second liquid delivery line, the second pump is provided at a position closer to the first reservoir tank than to the confluence site joined with the first liquid delivery line.
-Item 3-The device recited in Item 1 or 2, wherein
   the hydrocyclone is configured to separate a liquid, which has been sent to the hydrocyclone, into an A liquid and a B liquid,
   the hydrocyclone comprises a first derivation port that derives the A liquid and comprises a second derivation port that derives the B liquid,
   the first derivation port is connected to the first liquid delivery line, and
   the first pump is provided between the first derivation port and the confluence site joined with the second liquid delivery line.
-Item 4-The device recited in any one of Items 1 to 3, wherein
   the device is used for purifying an antibody produced from a cultured cell.
-Item 5-The device recited in any one of Items 1 to 4, wherein
   the protein is an antibody, and
   an antibody binding protein, which is a ligand, is used for purifying the antibody.
-Item 6-A method for producing a purified protein, comprising:
   using the device recited in any one of Items 1 to 5 so as to purify a protein from the culture liquid of the cell.

Above, the specific example of the present disclosure has been described in detail, but these descriptions are merely illustrative and are not construed as limiting the scope of the appended claims. The technique recited in claims contains matters in which the above-illustrated specific example is variously deformed or changed.

### [Reference Signs List]

- 100: Device
- 110: Hydrocyclone
- 120: First reservoir tank
- 130: First pump
- 140: Second pump
- 150: Inline mixer

- 1: Device
- 1A: Reaction module
- 1B: First washing module
- 1C: Elution module
- 1D: Second washing module
- 1E: Equilibration module

## Claims

1. A device that purifies a protein from a culture liquid of a cell, comprising:
a hydrocyclone;
a first reservoir tank that retains a first liquid;
a first pump; and
a second pump,
wherein
the first pump is provided at a site where a flow rate of a liquid discharged from the hydrocyclone can be adjusted, and
the second pump is provided at a site where a flow rate of the first liquid discharged from the first reservoir tank can be adjusted.

2. The device according to claim 1, further comprising:
an inline mixer that is positioned at a downstream of the hydrocyclone;
a first liquid delivery line that connects the hydrocyclone and the inline mixer; and
a second liquid delivery line that extends from the first reservoir tank and that is joined to the first liquid delivery line,
wherein
on the first liquid delivery line, the first pump is provided at a position closer to the hydrocyclone than to a confluence site joined with the second liquid delivery line, and
on the second liquid delivery line, the second pump is provided at a position closer to the first reservoir tank than to the confluence site joined with the first liquid delivery line.

3. The device according to claim 2, wherein
the hydrocyclone is configured to separate a liquid, which has been sent to the hydrocyclone, into an A liquid and a B liquid,
the hydrocyclone comprises a first derivation port that derives the A liquid and comprises a second derivation port that derives the B liquid,
the first derivation port is connected to the first liquid delivery line, and
the first pump is provided between the first derivation port and the confluence site joined with the second liquid delivery line.

4. The device according to any one of claims 1 to 3, wherein
the device is used for purifying an antibody produced from a cultured cell.

5. The device according to claim 4, wherein
the protein is an antibody, and
an antibody binding protein, which is a ligand, is used for purifying the antibody.

6. A method for producing a purified protein, comprising:
using the device according to any one of claims 1 to 3 so as to purify a protein from the culture liquid of the cell.
